# EUROPEAN PATENT APPLICATION

(11) **EP 4 269 619 A1**
(43) Date of publication of application: **01.11.2023**
(21) Application number: 21923376.4
(22) Date of filing: 20.10.2021
(51) Int. Cl.: C12Q 1/6883, C12Q 1/689

(54) **COMPOSITION FOR DIAGNOSING LYME DISEASE AND KIT COMPRISING SAME**

(30) Priority: 26.01.2021 KR 20210010808
(71) Applicant: Aithenutrigene Co., Seongnam-si, Gyeonggi-do 13449 (KR)
(72) Inventor: KIM, Jong Chul, Seongnam-si, Gyeonggi-do 13441 (KR)
(74) Representative: BCKIP Part mbB
(86) International application number: PCT/KR2021/014692
(87) International publication number: WO 2022/163967

(57) **Abstract**

The present invention relates to a primer set for diagnosing Lyme disease and a composition comprising the same. Specifically, using a composition and kit comprising the primer set capable of detecting the causative agent of Lyme disease, it is possible to detect a Borrelia strain, which is the causative agent of Lyme disease, in a more accurate manner, and to diagnose Lyme disease in a rapid and inexpensive manner.

## Description

### Technical Field

The present invention relates to a composition for diagnosing Lyme disease and a composition for detecting the same. Specifically, using a primer set comprised in the composition of the present invention, it is possible to detect a *Borrelia* strain, which is the causative agent of Lyme disease, in a more accurate manner, and to diagnose Lyme disease in a rapid and inexpensive manner.

### Background Art

Lyme disease is an infectious disease in which the spiral bacterium *Borrelia* invades the body when a tick which is an insect bites a person, causing the disease in various organs. It is known that, in the early stage of the disease, erythema migrans, a characteristic skin lesion, appears along with fever, headache, and fatigue. Erythema migrans is a skin lesion showing a characteristic shape that is red at the edges and pale in the middle, like a bull's eye. If not treated, the bacterium spreads to various organs after several days to weeks, causing encephalitis, peripheral neuritis, myocarditis, arrhythmia, and musculoskeletal pain. If the disease is not treated appropriately with antibiotics at an early stage, it becomes chronic and difficult to treat. In this case, despite antibiotic treatment, fatigue, musculoskeletal pain, and neurologic symptoms may persist for years, and in rare cases, may result in death. Therefore, it is important to diagnose Lyme disease at an early stage.

Meanwhile, the bacterium *Borrelia*, which is the causative agent of Lyme disease, is also a causative agent of relapsing fever, which shows initial symptoms similar to those of Lyme disease. Relapsing fever is characterized in that chronic erythema migrans does not appear, unlike Lyme disease. However, diagnosing Lyme disease while observing the manifestation of these symptoms not only causes pain to the patient, but also may miss the appropriate treatment timing. Therefore, there is a need for means for rapidly and accurately diagnosing Lyme disease.

### DISCLOSURE

### Technical Problem

An object of the present invention is to provide a primer set for diagnosing Lyme disease.

Another object of the present invention is to provide a composition for diagnosing Lyme disease comprising the primer set.

Still another object of the present invention is to provide a kit for diagnosing Lyme disease comprising the primer set.

Yet another object of the present invention is to provide a method of diagnosing Lyme disease from a biological sample using the primer set.

### Technical Solution

One embodiment for achieving the above objects provides a primer set for diagnosing Lyme disease comprising six primers. The primer set consisting of six primers may be selected from among a primer set represented by the nucleotide sequences of SEQ ID NOs: 1 to 6, a primer set represented by the nucleotide sequences of SEQ ID NOs: 7 to 12, and a primer set represented by the nucleotide sequences of SEQ ID NOs: 13 to 18.

The primer set is for diagnosing Lyme disease, and more specifically, may be for detecting a *Borellia* sp. strain, which is the causative agent of Lyme disease.

The *Borrelia* strain, which is the causative agent of Lyme disease, may be selected from the group consisting of *Borrelia afzelii*, *Borrelia burgdorferi*, and *Borrelia garinii.*

The term "primer" refers to a short polynucleotide having a free 3'-hydroxyl group, which is capable of forming a base pair with a complementary template and serving as a starting point for template strand copying. A primer is able to initiate DNA synthesis in the presence of a reagent for polymerization (DNA polymerase or reverse transcriptase) and four different deoxynucleoside triphosphates (dNTPs) in a suitable buffer and at a suitable temperature.

Each primer in the primer set is specific to the target gene, and the term "specific" or "complementary" to the gene means that the primer is able to at least 80%, at least 85%, at least 90%, at least 95%, or 100% base-pair with the target gene.

Each primer in the primer set may consist of or comprise one of the nucleotide sequences of SEQ ID NOs: 1 to 18, and may be at least 80%, at least 90%, at least 95%, at least 97%, or at least 99% identical to one of the nucleotide sequences of SEQ ID NOs: 1 to 18 as long as it binds specifically to the genetic material of the causative agent of Lyme disease. The primers may also be modified to comprise additional features within a range that does not change the basic properties required to act as a starting point for DNA synthesis. Thus, the primer may further comprise an additional nucleotide sequence of about 1 to 20 bp, 1 to 15 bp, 1 to 10 bp, or 1 to 5 bp at the 3' end, the 5' end or inside the nucleotide sequence of the primer, while comprising the nucleotide sequence of each of SEQ ID NOs: 1 to 18, and if necessary, may further comprise, at each end or a specific nucleotide, a label that is detectable directly or indirectly by spectroscopic, photochemical, biochemical, immunochemical, or chemical means.

The label may be, for example, a chromogenic enzyme, a fluorescent substance, a radioactive isotope, or a colloid. The chromogenic enzyme may be peroxidase, alkaline phosphatase, or acidic phosphatase, the fluorescent substance may be thiourea (FTH), 7-acetoxycoumarin-3-yl, fluorescein-5-yl, fluorescein-6-yl, 2',7'-dichlorofluorescein- 5-yl, 2',7'-dichlorofluorescein-6-yl, dihydrotetramethylrosamine-4-yl, tetramethylrhodamine-5-yl, tetramethylrhodamine-6-yl, 4,4-difluoro-5,7-dimethyl-4-bora-3a,4a-diaza-s-indacene-3-ethyl or 4,4-difluoro-5,7-diphenyl-4-bora-3a,4a-diaza-s-indacene-3-ethyl, Cy3, Cy5, poly L-lysine-fluorescein isothiocyanate (FITC), rhodamine-B-isothiocyanate (RITC), phycoerythrin (PE), or rhodamine, and the radioactive isotope may be 32P, without being limited thereto.

The primer set of the present invention may be suitable for use in isothermal amplification. The term "isothermal amplification" refers to amplification of DNA under a single temperature condition. Unlike a conventional polymerase chain reaction (PCR) which should be performed using a specific PCR system as it requires different temperatures and conditions for denaturation, annealing, and extension steps, the isothermal amplification reaction has the advantage of not requiring a separate special device. In general, the isothermal amplification method is able to amplify up to 10⁹ target nucleic acids within 1 hour in a specific temperature range, and may be performed with a constant-temperature device capable of maintaining the temperature. In addition, the isothermal amplification method is performed within a short time and the result may be observed with the naked eye, and thus amplification may be easily confirmed.

In one embodiment, the isothermal amplification may be loop-mediated isothermal amplification. The term "loop-mediated isothermal amplification (LAMP)" refers to an amplification method that is performed isothermally using outer primers, inner primers, and additional loop primers to enhance the reaction rate. Among the primers used in the LAMP reaction, the outer primers consist of two types of primers (forward outer (F3) primer and backward outer (B3) primer), and serve to unwind DNA double strands during the non-cyclic step of the reaction. The inner primers consist of two types of primers (forward inner primer (FIP) and backward inner primer (BIP)), and are composed of nucleotides corresponding to the forward and backward nucleotide sequences so as to form loops essential for loop-mediated isothermal amplification reaction. These inner primers are not included in the genetic material of the strain, but may be designed to further include specific bases, for example, a series of thymine bases, to increase the efficiency of the reaction. The two additional primers consist of two types of primers (forward loop (LoopF) primer and backward loop (LoopB) primer), and adhere to a nucleotide sequence (ring site) to which the inner primers do not bind, thereby accelerating the ring-mediated isothermal amplification reaction. In the loop-mediated isothermal amplification method, the inner primers are bound to a template and extended, and then the outer primers are bound to the outside of each DNA helix and extended. At this time, strand displacement occurs due to extension of the outer primers, and accordingly, the first synthesized strand is detached from the template, and the detached synthetic strand forms a loop structure from the 5' end, and then a loop structure is formed at the 3' end in the same process, and an amplification reaction occurs through these loops. The isothermal amplification or loop-mediated isothermal amplification reaction may be a reverse transcription reaction.

The isothermal amplification reaction may be performed at 50 to 70°C, 52 to 70°C, 52 to 68°C, 52 to 65°C, 55 to 65°C, or 50 to 60°C or 65°C. In addition, the isothermal amplification method may be performed for 10 to 90 minutes, 20 to 90 minutes, 25 to 90 minutes, 10 to 80 minutes, 20 to 80 minutes, 25 to 80 minutes, 10 to 70 minutes, 20 to 70 minutes, 25 to 70 minutes, 10 to 60 minutes, 20 to 60 minutes, 25 to 60 minutes, or 30 to 40 minutes.

In one embodiment, using the primer set, it is possible to detect the causative agent of Lyme disease in a sample containing a target gene such as 100 copies or more, or 500 copies or more of DNA or RNA per 20 µl of a reaction solution. In addition, it is possible to detect the causative agent of Lyme disease within a short time under general isothermal amplification reaction conditions. For example, it is possible to perform an isothermal amplification reaction using 0.2 to 1.6 µM of each primer of the primer set at 60 to 65°C, specifically 65°C, for about 40 minutes, for example, 30 minutes to 60 minutes, 30 minutes to 50 minutes, or 30 minutes to 40 minutes, and to confirm the detection result. In the primer set, each primer may be preferably used at 1.6 µM for each of the FIP and BIP primers, 0.2 µM for each of the F3 and B3 primers, and 0.4 µM for each of the FL and BL primers. In the isothermal amplification reaction, results should be produced within a short time in a limited environment which is isothermal, and thus the combination of primers and the ratio between components are important factors to obtain significant reaction results.

Another embodiment of the present invention includes a composition or kit for diagnosing Lyme disease comprising the primer set.

The composition or kit for diagnosing Lyme disease may further comprise appropriate reactants and reagents for performing an isothermal amplification reaction. These reactants and reagents may include, for example, additional primers, dNTPs, and enzymes (DNA polymerase or reverse transcriptase), and may further include inorganic salts such as potassium chloride, magnesium sulfate, and ammonium sulfate, or a surfactant, to increase the efficiency of the reaction. In one embodiment, the reactants and reagents may be a solution (pH 7 to 9, preferably pH 8.8) containing 10 to 20 mM Tris-HCl, 1 to 10 mM (NH₄)₂SO₄, 10 to 50 nM KCl, 0.1 to 2 mM MgSO₄, and 0.01 to 0.1% Tween-20, more specifically 20 mM Tris-HCl, 10 mM (NH₄)₂SO₄, 50 nM KCl, 2 mM MgSO₄, and 0.1% Tween-20.

In one embodiment, the composition or kit for diagnosing Lyme disease may further comprise reagents enabling a reaction result to be visually confirmed. Such reagents may be commonly known or commercially available reagents, and examples thereof include, but are not limited to, WarmStart^{®} LAMP Mix. These reagents may include Tris-HCl, (NH₄)₂SO₄, KCl, MgSO₄, and Tween-20, as mentioned above, so that the reaction occurs efficiently. In addition, these reagents may further include an enzyme for performing an amplification reaction, such as DNA polymerase or reverse transcriptase.

The composition or kit for diagnosing Lyme disease may further comprise a negative control material or a positive control material to accurately read the diagnosis result. As the negative control material, template RNA or DNA unrelated to the causative agent of Lyme disease may be used, and as the positive control material, either a biological sample obtained from a patient infected with the causative agent of Lyme disease or a template RNA or DNA corresponding to a part of the genome of the causative agent of Lyme disease may be used. These control materials may be applied to the gene amplification reaction separately from or together with the biological sample in one tube, as needed.

Still another embodiment of the present invention provides a method for diagnosing Lyme disease comprising steps of: mixing the primer set with a biological sample; and amplifying a gene by subjecting the mixture to an isothermal amplification reaction.

The biological sample is obtained from a subject to be tested, and examples thereof include a sample from the nasopharynx or oropharynx through swab or nasal washing, bronchial lavage or aspirate, intratracheal aspirate and bronchial biopsy, sputum, bronchoalveolar lavage, saliva, blood, urine, feces, and the like.

The biological sample may be pretreated to increase the efficiency of the amplification reaction. The pretreatment may be performed by diluting the biological sample 10-fold, 50-fold, 100-fold, 200-fold, or 400-fold with water; or heat-treating the biological sample after treatment with proteinase K; or extracting a gene from the biological sample or purifying the gene to a certain level, by using a commonly known method such as anion exchange chromatography, affinity chromatography, size exclusion chromatography, liquid chromatography, continuous extraction, centrifugation, or gel electrophoresis, or using a commercially available kit. When RNA is extracted from the biological sample and used, the method may further comprise a step of subjecting the RNA to reverse transcription into cDNA, before amplifying the target gene. The pretreatment may mean treating the biological sample with a lysis buffer containing 50 mM Tris-HCl (pH 8.0), 150 mM NaCl, 5 mM EDTA, and 1% NP-40, or a solution containing 0.1 to 0.5 w/w% Brij-35, 10 mM to 300 mM Tris-HCl (pH 7.0 to 8.0), and 1 mM to 10 mM CaCl₂, specifically 0.3 w/w% Brij-35, 100 mM Tris-HCl (pH 7.5), and 5 mM CaCl₂. Specifically, the pretreatment may mean treating the biological sample with the solution and then obtaining only the gene from the sample by purification according to a conventional method. The combination of the pretreatment solutions may facilitate extraction of the gene from the sample and enhance the isothermal amplification reaction efficiency of the primer set of the present invention.

The method for diagnosing Lyme disease comprises a step of detecting the amplified gene. Detection of the amplification product may be performed through fluorescence measurement using SYBR Green I, color change of an indicator such as phenol red, turbidity, precipitate formation, DNA laddering, capillary electrophoresis, DNA chip assay, gel electrophoresis, radioactivity measurement, or phosphorescence measurement, without being limited thereto.

The kit of the present invention may further comprise a container containing each reaction reagent, a tool for use in the test, and a user guide describing optimal conditions for performing the reaction.

### Advantageous Effects

Using the primer set of the present invention, it is possible to specifically detect Lyme disease, and to detect Lyme disease *in situ* in a rapid and accurate manner using an isothermal amplification reaction.

In addition, since the isothermal amplification reaction does not require expensive equipment and specialized personnel that are required to meet the specific temperature and conditions for performing the amplification reaction, it may be performed efficiently and at low cost, and thus makes it possible to diagnose a large number of test subjects at early stages.

### Best Mode

The present invention is directed to a primer set for diagnosing Lyme disease selected from the group consisting of a primer set represented by the nucleotide sequences of SEQ ID NOs: 1 to 6, a primer set represented by the nucleotide sequences of SEQ ID NOs: 7 to 12, and a primer set represented by the nucleotide sequences of SEQ ID NOs: 13 to 18.

### Mode for Invention

Hereinafter, the present invention will be described in detail with reference to examples to aid understanding of the present invention. However, the examples according to the present invention may be modified into a variety of different forms, and the scope of the present invention should not be construed as being limited to the following examples. The examples of the present invention are provided in order to more completely explain the present invention to those skilled in the art.

### Example 1: Construction of primers

To detect the causative agent of Lyme disease using a loop-mediated isothermal amplification (LAMP), primers were designed and constructed. In order to obtain primer set candidates, nucleotide sequences were confirmed through blast analysis between 52 *Borrelia* strains, and among them, primer sequence candidates targeting *Borrelia afzelii*, *Borrelia burgdorferi*, and *Borrelia garinii,* known as the causative agents of Lyme disease, were selected. The selected primer sequences were tested probabilistically using a program to see if there was no cross-reaction with other *Borrelia* strains. Then, primer candidates with low cross-reactivity were screened for 15 *Borrelia* strains that were not the causative agents of Lyme disease among the 52 *Borrelia* strains, and actual primer sets with low cross-reactivity were selected. The selected primers are shown in Table 1 below. The following primers included outer primers, inner primers, and loop primers, and four thymines were incorporated into the FIP primer and the BIP primer to promote loop formation and increase the reaction rate.

**[Table 1]**

| | Primer type | Sequence |
|---|---|---|
| Primer set 1 (targeting *B. afzelii*) | F3 | 5'-GGTATACTGACAGCAGCTT-3' (SEQ ID NO: 1) |
| | B3 | 5'-CTTGCAGCTTAATAATAGCCTT-3' (SEQ ID NO: 2) |
| | FIP | 5'- GTTGCTCGGTCCTCCATGTTTAAATTTTTAATGTTATCCG TGATATGGTTCCGA-3' (SEQ ID NO: 3) |
| | BIP | 5'- GGATTTCGTATCAATTTTGGAGGCATTTTAAGTTACAAA |
| | | GGTCCCATTGC-3' (SEQ ID NO: 4) |
| | FL | 5'-ATAAGGCCTTCGGTATTG-3' (SEQ ID NO: 5) |
| | BL | 5'-ATTCTACGTTCCGATTCTCAGTAT-3' (SEQ ID NO: 6) |
| Primer set 2 (targeting *B. burgdorferi*) | F3 | 5'-AGAGCAGCTGAGGAGCT-3' (SEQ ID NO: 7) |
| | B3 | 5'-CTTCCAGTTGAACACCATCTT-3' (SEQ ID NO: 8) |
| | FIP | 5'- AAGTCCACGACGGTTGAGACCTTTTTGCAGCCTGCTTAA ATTAACA-3' (SEQ ID NO: 9) |
| | BIP | 5'- GAGCAAACGAAGTTGAAGCTATTTTAGCCTGAGCAGTT AGAGC-3' (SEQ ID NO: 10) |
| | FL | 5'-TGAATGCGATCCTGGT-3' (SEQ ID NO: 11) |
| | BL | 5'-TATGGAGCTAATGTTGCTAAT-3' (SEQ ID NO: 12) |
| Primer set 3 (targeting *B. garinii*) | F3 | 5'-GCATCGTTGACGTCAGCT-3' (SEQ ID NO: 13) |
| | B3 | 5'-TGTAGAGGCGATTGTAGC-3' (SEQ ID NO: 14) |
| | FIP | 5'- CCACGATTACTTGCTGGTAACTTTTGTGACGTGTTCGGT TAAGT-3' (SEQ ID NO: 15) |
| | BIP | 5'- GATAAGAGTGCCGCTGATAAGTTTTTAGCCCAGCAGAT AAGGG-3' (SEQ ID NO: 16) |
| | FL | 5'-TAACAACGGTTGCGGTCG-3' (SEQ ID NO: 17) |
| | BL | 5'-TGACGATGAGGTCAATTCAT-3' (SEQ ID NO: 18) |

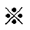 F3, forward primer; B3, backward primer; FIP, forward inner primer; BIP, backward inner primer; FL, forward loop primer; BL, backward loop primer

### Test Example 1: Evaluation of analytical performance

### (1) Preparation of isothermal amplification reagents

Each primer set in Table 1 above was prepared as a primer mix having the composition and concentration shown in Table 2 below. *Borrelia afzeli, Borrelia burgdorferi*, and *Borrelia garinii* strains, which are the causative agents of Lyme disease, were obtained, and each of the strains was treated with lysis buffer (50 mM Tris-HCl (pH 8.0), 150 mM NaCl, 5 mM EDTA, and 1% NP-40) and centrifuged. Then, the supernatant was collected, and the genetic material was extracted therefrom using an RNA extraction kit (Qiagen) and then dissolved in purified water. The concentration was equalized by measuring the absorbance, and the resulting RNA was used in the following test examples. 2 µl of 10X primer mix, 10 µl of colorimetric LAMP 2X master mix (WarmStart^{®} Inc.), and 7 µl of dH₂O were mixed with 1 µl of the RNA, thus preparing a total of 20 µl of a mixed solution. The genetic material of the strain was not added to the control group. The mixed solution has a composition shown in Table 3 below. The prepared mixed solution was subjected to an isothermal amplification reaction at 65°C for 40 minutes, and then the reaction was stopped and the color change was visually observed.

**[Table 2]**

| Primer | 10X concentration (stock) (µM) | 1X concentration (final) (µM) |
|---|---|---|
| FIP | 16 | 1.6 |
| BIP | 16 | 1.6 |
| F3 | 2 | 0.2 |
| B3 | 2 | 0.2 |
| FL | 4 | 0.4 |
| BL | 4 | 0.4 |

**[Table 3]**

| | DNA-targeting detection group | Control group |
|---|---|---|
| Colorimetric LAMP 2X master mix | 10 µl | 10 µl |
| Primer mix (10X) | 2 µl | 2 µl |
| Target DNA | 1 µl | 0 |
| dH₂O | 7 µl | 8 µl |
| Total volume | 20 µl | 20 µl |

### (2) Evaluation of analysis performance

Using the three primer sets in Table 1 above, the analytical performance of the primer set according to the present invention was evaluated.

Specifically, the RNA from each of *Borrelia afzelii, Borrelia burgdorferi*, and *Borrelia garinii* strains was diluted serially, thus preparing reaction solutions containing 100, 500, and 1,000 copies per 20 µl of the reaction solution, and each reaction solution was subjected to an isothermal amplification reaction. For each strain, the primer set targeting the strain was used. Thereafter, the color change was visually observed, and the result was confirmed through electrophoresis. If the color of the reaction product remained pink, it was judged as negative (-), and if the color changed to yellow, it was judged as positive (+). In addition, the amplification result was directly confirmed by electrophoresis.

As a result, as shown in Table 4 below, it was confirmed that, for all of the strains, the color of the reaction product changed to yellow from 100 or 500 copies, indicating that the genetic material was amplified.

**[Table 4]**

| Strain | Primer | Untreated | 100 copies | 500 copies | 1,000 copies |
|---|---|---|---|---|---|
| *Borrelia afzelii* | Set 1 | - | - | + | + |
| *Borrelia burgdorferi* | Set 2 | - | + | + | + |
| *Borrelia garinii* | Set 3 | - | + | + | + |

### Test Example 2: Evaluation of analytical performance depending on reaction time

Using the three primer sets in Table 1 above, the reaction time-dependent analytical performance of the primer set according to the present invention was evaluated.

Specifically, using Test Example 1 and 500 copies/20 µl of RNA of each strain as a template, mixed solutions for amplification reaction were prepared as described in Test Example 1, and subjected to an isothermal amplification reaction for 10 minutes, 20 minutes, 30 minutes, 40 minutes, and 60 minutes. Thereafter, the color change was visually observed and the result was confirmed through electrophoresis. If the color of the reaction product remained pink, it was judged as negative, and if the color changed to yellow, it was judged as positive.

As a result, as shown in Table 5 below, it was confirmed that positive results were observed from 20 minutes, and the color of all reaction products changed to yellow after 40 minutes, indicating that the genetic material was amplified.

**[Table 5]**

| Strain | Primer | Untreated (60 min) | 10 min | 20 min | 30 min | 40 min | 60 min |
|---|---|---|---|---|---|---|---|
| *Borrelia afzelii* | Set 1 | - | - | - | + | + | + |
| *Borrelia burgdorferi* | Set 2 | - | - | - | + | + | + |
| *Borrelia garinii* | Set 3 | - | - | + | + | + | + |

### Test Example 3: Evaluation of analysis specificity

Using the three primer sets in Table 1 above, analysis specificity of the primer set of the present invention for each strain was evaluated.

As comparison groups for this evaluation, *Borrelia recurrentis, Borrelia crocidurae*, and *Borrelia dutonii*, known as the causative agents of relapsing fever, among *Borrelia* strains, were used. RNA from each strain was prepared as described in Test Example 1 and used as a template. Using 500 copies/20 µl of RNA, obtained from each of the target strains *Borrelia afzelii, Borrelia burgdorferi*, and *Borrelia garini* and the comparison group strains, and each of the primer sets shown in Table 1 above, mixed solutions for amplification reaction were prepared as described in Test Example 1 above, and the comparison groups were also subjected to an isothermal amplification reaction for 60 minutes to guarantee sufficient reaction. RNA from the target strain of each primer set was used as a positive control group. The color change of the reaction product was observed visually, and classified into negative (-) as pink and positive (+) as yellow, and the result was verified again by electrophoresis.

As a result, as shown in Table 6 below, all of the primer sets in Table 1 exhibited a color change to yellow and gene amplification for the red strains, but exhibited no color change in the comparison groups, even though the comparison groups were subjected to the isothermal amplification reaction for 60 minutes.

Therefore, it was confirmed that the primer set of the present invention had specificity only for the causative agent of Lyme disease, indicating that the primer set is suitable for diagnosing Lyme disease differentially from relapsing fever.

**[Table 6]**

| | Positive control | *Borrelia recurrentis* | *Borrelia crocidurae* | *Borrelia dutonii* |
|---|---|---|---|---|
| Primer set 1 | + | - | - | - |
| Primer set 2 | + | - | - | - |
| Primer set 3 | + | - | - | - |

### Test Example 4: Evaluation of analytical performance using virtual sample

In order to evaluate whether analysis can be performed on actual clinical samples, virtual samples were prepared and used to evaluate the analytical performance of the primer set according to the present invention.

Specifically, each of blood samples from 40 healthy people who have never been bitten by a tick or suffering from Lyme disease was divided into eight groups. Then, as shown in Table 7 below, seven of the eight groups were mixed with each or a combination of the genetic materials of the 3 causative agents of Lyme disease at 500 copies per 20 µl of the blood sample, thus preparing virtual patient samples, and one group was used as an untreated negative control group. Mixed solutions for amplification reaction were prepared using the above samples and subjected to an isothermal amplification reaction.

The results are shown in Table 7 below.

**[Table 7]**

| | Number of samples | Primer set 1 (targeting *B. afzerii*) | Primer set 2 (targeting *B*. *burgdorferi*) | Primer set 3 (targeting *B. garinii*) |
|---|---|---|---|---|
| *B. afzerii* | 30 | 30 | 0 | 0 |
| *B. burgdorferi* | 30 | 0 | 30 | 0 |
| *B. garinii* | 30 | 0 | 0 | 29/30 |
| *B. afzerii* + *B. burgdoferi* | 30 | 29/30 | 30 | 0 |
| *B. afzerii* + *B. garinii* | 30 | 30 | 0 | 30 |
| *B. burgdoferi* + *B. garinii* | 30 | 0 | 29/30 | 30 |
| *B. afzerii* + *B. burgdorferi* +*B. garinii* | 30 | 29/30 | 30 | 29/30 |
| Negative control group | 10 | 0 | 0 | 0 |

### Test Example 5: Comparison of analytical performance between isothermal amplification reaction and PCR

The analytical performance of the isothermal amplification reaction using the primers of the present invention was evaluated comparatively with that of PCR.

Specifically, samples were randomly selected among the samples of Test Example 4, and each sample was divided into two groups. Then, one of the two groups was mixed with 500 copies/20 µl of the genetic material from *Borrelia burgdorferi* as described in Test Example 4 above, and the other group was prepared as a sample without any treatment. Thereafter, the same isothermal amplification reaction as described in Test Example 1 was performed on each sample using primer set 2. Meanwhile, reagents shown in Table 8 below were prepared using the forward primer (F3) and reverse primer (B3) of primer set 2 and subjected to PCR under the conditions shown in Table 9 below. The results are shown in Table 10 below as the number of positive samples per the total number of samples.

**[Table 8]**

| | Strain-treated group | Untreated group |
|---|---|---|
| 2X PCR master mix (Quiagen) | 10 µl | 10 µl |
| Forward primer (F3, 10 pmole) | 1 µl | 1 µl |
| Backward primer (B3, 10 pmole) | 1 µl | 1 µl |
| Target DNA | 1 µl | 0 |
| H₂O | 7 µl | 8 µl |
| Total volume | 20 µl | 20 µl |

**[Table 9]**

| | Temperature | Time | Number of cycles |
|---|---|---|---|
| Heat-inactivation | 95°C | 2 min | 1 |
| Denaturation) | 95°C | 10 sec | 45 |
| Annealing) | 60°C | 10 sec | |
| Extension) | 72°C | 20 sec | |
| Cooling) | 40°C | 30 sec | 1 |

**[Table 10]**

| Analysis method | Strain-treated group | Untreated group | Sensitivity/specificity (%) |
|---|---|---|---|
| LAMP | 29/30 | 0/30 | 96.7/100 |
| PCR | 27/30 | 2/30 | 90.0/93.3 |

Therefore, it was confirmed that isothermal amplification reaction using the primers of the present invention had better sensitivity and specificity than simple PCR.

### Industrial Applicability

The present invention relates to a composition for diagnosing Lyme disease and a composition for detecting the same. Specifically, using a primer set comprised in the composition of the present invention, it is possible to detect a *Borrelia* strain, which is the causative agent of Lyme disease, in a more accurate manner, and to diagnose Lyme disease in a rapid and inexpensive manner.

## Claims

1. A primer set for diagnosing Lyme disease selected from the group consisting of a primer set represented by the nucleotide sequences of SEQ ID NOs: 1 to 6, a primer set represented by the nucleotide sequences of SEQ ID NOs: 7 to 12, and a primer set represented by the nucleotide sequences of SEQ ID NOs: 13 to 18.

2. The primer set of claim 1, which is able to diagnose Lyme disease by performing loop-mediated isothermal amplification.

3. The primer set of claim 1, which is for detecting a strain selected from the group consisting of *Borrelia afzelii*, *Borrelia burgdorferi*, and *Borrelia garinii.*

4. A composition for diagnosing Lyme disease comprising at least one primer set selected from the group consisting of a primer set represented by the nucleotide sequences of SEQ ID NOs: 1 to 6, a primer set represented by the nucleotide sequences of SEQ ID NOs: 7 to 12, and a primer set represented by the nucleotide sequences of SEQ ID NOs: 13 to 18.

5. A kit for diagnosing Lyme disease comprising the composition of claim 4.

6. A method for diagnosing Lyme disease comprising steps of:
mixing the composition of claim 4 and a biological sample; and
amplifying a gene by subjecting the mixture to an amplification reaction.

7. The method of claim 6, wherein the amplification is isothermal amplification.


### Sequence Listing Free Test

SEQUENCE LISTING
<110>    NUTRIGENE
<120>    Composition for diagnosis of Lyme disease and kit comprising the same
<130> P2021-0150
<160> 18
<170> PatentIn version 3.2
<210> 1
<211> 19
<212> DNA
<213> Artificial
<220><223> B.Afzelii forward primer
<400> 1
ggtatactga cagcagctt            19
<210> 2
<211> 22
<212> DNA

<213> Artificial

<220><223> B.Afzelii backward primer

<400> 2

cttgcagctt aataatagcc tt          22

<210> 3

<211> 54

<212> DNA

<213> Artificial

<220><223> B.Afzelii forward inner primer

<400> 3

gttgctcggt cctccatgtt taaattttta atgttatccg tgatatggtt ccga          54

<210> 4

<211> 50

<212> DNA

<213> Artificial

<220><223> B.Afzelii backward inner primer

<400> 4

ggatttcgta tcaattttgg aggcatttta agttacaaag gtcccattgc          50

<210> 5

<211> 18

<212> DNA

<213> Artificial

<220><223> B.Afzelii forward loop primer

<400> 5

ataaggcctt cggtattg          18

<210> 6

<211> 24

<212> DNA

<213> Artificial

<220><223> B.Afzelii backward loop primer

<400> 6

attctacgtt ccgattctca gtat 24

<210> 7

<211> 17

<212> DNA

<213> Artificial

<220><223> B.Burgdorferi forward primer

<400> 7

agagcagctg aggagct 17

<210> 8

<211> 21

<212> DNA

<213> Artificial

<220><223> B.Burgdorferi backward primer

<400> 8

cttccagttg aacaccatct t 21

<210> 9

<211> 46

<212> DNA

<213> Artificial

<220><223> B.Burgdorferi forward inner primer

<400> 9

aagtccacga cggttgagac ctttttgcag cctgcttaaa ttaaca 46

<210> 10

<211> 43

<212> DNA

<213> Artificial

<220><223> B.Burgdorferi backward inner primer

<400>10

gagcaaacga agttgaagct attttagcct gagcagttag agc 43

<210> 11

<211> 16

<212> DNA

<213> Artificial

<220><223> B.Burgdorferi forward loop primer

<400> 11

tgaatgcgat cctggt 16

<210> 12

<211> 21

<212> DNA

<213> Artificial

<220><223> B.Burgdorferi backward loop primer

<400> 12

tatggagcta atgttgctaa t 21

<210> 13

<211> 18

<212> DNA

<213> Artificial

<220><223> B. garinii forward primer

<400> 13

gcatcgttga cgtcagct 18

<210> 14

<211> 18

<212> DNA

<213> Artificial

<220><223> B. garinii backward primer

<400> 14

tgtagaggcg attgtagc 18

<210> 15

<211> 44

<212> DNA

<213> Artificial

<220><223> B. garinii forward inner primer

<400> 15

ccacgattac ttgctggtaa cttttgtgac gtgttcggtt aagt 44

<210> 16

<211> 43

<212> DNA

<213> Artificial

<220><223> B. garinii backward inner primer

<400> 16

gataagagtg ccgctgataa gtttttagcc cagcagataa ggg 43

<210> 17

<211> 18

<212> DNA

<213> Artificial

<220><223> B. garinii forward loop primer

<400> 17

taacaacggt tgcggtcg 18

<210> 18

<211> 20

<212> DNA

<213> Artificial

<220><223> B. garinii backward loop primer

<400> 18

tgacgatgag gtcaattcat 20